# EUROPEAN PATENT SPECIFICATION

(11) **EP 3 289 405 B1**
(45) Date of publication and mention of the grant of the patent: **30.06.2021**
(21) Application number: 16785982.6
(22) Date of filing: 29.04.2016
(51) Int. Cl.: G02C 7/04, A61B 3/16

(54) **CONTACT LENS WITHOUT MOIRE PATTERN IN CENTER**
KONTAKTLINSE OHNE MOIRE-MUSTER IN DER MITTE
LENTILLE DE CONTACT SANS MOTIF DE MOIRÉ DANS LE CENTRE

(30) Priority: 30.04.2015 US 201562155334 P; 01.05.2015 US 201562155747 P
(43) Date of publication of application: 07.03.2018
(73) Proprietor: Wang, Lon, Taipei 106, Taiwan (TW)
(72) Inventor: WANG, Lon, Taipei 106 (TW); HSIAU, Wei-Chung, Taipei 106 (TW)
(74) Representative: Viering, Jentschura & Partner mbB Patent- und Rechtsanwälte
(86) International application number: PCT/CN2016/080758
(87) International publication number: WO 2016/173554

(56) References cited:
- CN-A- 103 415 244
- US-A- 4 889 421
- US-A1- 2014 163 351

## Description

### BACKGROUND

### Technical Field

The present invention relates to a sensing technology for an intraocular pressure (IOP), and, in particular, relates to a contact lens without moire pattern in center, method for analyzing change of moire pattern thereof, and system for monitoring an intraocular pressure variation.

### Related Art

Intraocular pressure is one of the parameters for assessing the advancing of glaucoma, and an excessive change in intraocular pressure can cause damages to optic nerves. The variation of intraocular pressure is desired to be continually monitored so that an early detection of glaucoma and proper medical administration to slow down its progression can be made possible.

The most common manner for measuring intraocular pressure is by using a contact applanation tonometer, for example, a Goldmann tonometer. However, the continual use of a contact tonometer for measurement causes burden and inconvenience to a user, therefore a contact tonometer is not appropriate for long-term monitoring of intraocular pressure.

Since the change of intraocular pressure causes the change of cornea curvature, the change of intraocular pressure can be obtained through measuring the change of cornea curvature. Cornea curvature is generally obtained in the following manner: a device is used to project a stripe pattern onto a cornea; a recorder is used to record the image reflected from the surface of cornea; the amount of stripe pattern change in the reflected image is analyzed to find the curvature of cornea. During the measurement, the head of a user must be placed on the bracket, so that the eye of the user can be properly positioned. Then, a stripe pattern is projected to the cornea region. And, the instrument begins to record the amounts of changes in the stripe, and converts them to find the variations of cornea curvature. For such measurement, however, a professional person is required to assist in operating the instrument and in the operation process lest the head movement of the user may cause measurement errors. The measurement of cornea curvature in reflective manner may need complex calculation because of the constraints incurred from the weakly reflected images. Additionally, the instrument is often too large to carry, and therefore not suitable as a portable device for monitoring intraocular pressure.

Some further designs are known from US 2014/163351 A1 and US 4 889 421 A.

US 2014/163351 discloses a contact lens for sensing change of intraocular pressure (paragraph 39), comprising:
- a first material layer (110) with a central region (112) with a first pattern (130) and a peripheral region (114) without a pattern (paragraphs 40-41);
- a second material layer (120) with a central region (122) with a second pattern (132) and a peripheral region (124) (paragraphs 44-46);
- a buffer layer (15) sandwiched between the first and second material layer (paragraph 48).

### SUMMARY

According to the present invention, a contact lens as defined by claim 1 is provided. The dependent claims show some examples thereof.

In one embodiment, a contact lens includes a first material layer and a first pattern. The first material layer has a central region and an annular region around the central region. When the contact lens is placed on a surface of an eyeball, the central region corresponds to a pupil of the eyeball and the annular region corresponds to an iris of the eyeball. The first pattern is formed in the annular region, and is formed of a plurality of first sub-patterns arranged at intervals. The first pattern is configured to overlap with a second pattern to form a moire pattern.

As above, according to the embodiments, the contact lens is designed to appear a moire pattern in the annular region of the surface thereof, and not appear the moire pattern in the central region corresponding to the viewing field (i.e. the pupil of the eyeball). The contact lens is configured to sense the curvature of the cornea of the eyeball of the user, such that the change of the moire pattern is directly related to the IOP variation. Therefore, the contact lens does not affect the viewing transparency for the user who wears it for continual IOP monitoring, and the amount of data to be processed for obtaining the corresponding IOP variation is significantly reduced. According to some embodiments, the change of the cornea of the eyeball of the user is accurately measured without need of fluorescent agents, thereby avoiding damage or infection to the cornea. According to some embodiments, the structure (e.g. the grooves or the protrusions) or the prints of the pattern and the main body (i.e., the light transmissive material layer) of the contact lens are integrally formed, and the pattern (i.e. the first pattern and the second pattern) of the contact lens do not contact the eyeball, thereby not affecting the wearing comfort.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### BRIEF DESCRIPTION OF THE DRAWINGS

The present invention will become more fully understood from the detailed description given hereinbelow illustration only, and thus are not limitative of the present invention, and wherein:
Fig. 1 is a view illustrating a use state of a contact lens of a first embodiment according to the present invention;
Fig. 2 is a schematic view of the contact lens of the first embodiment according to the present invention;
Fig. 3 is an explosion view of the contact lens of the first embodiment according to the present invention;
Fig. 4 is a schematic view of the contact lens of a second embodiment according to the present invention;
Fig. 5 is a schematic view of the contact lens of a third embodiment according to the present invention;
Fig. 6 is an explosion view of the contact lens of the third embodiment according to the present invention;
Fig. 7 is a schematic view of a pattern of a first embodiment;
Fig. 8 is a schematic view of a pattern of a second embodiment;
Fig. 9 is a schematic view of a pattern of a third embodiment;
Fig. 10 is a schematic view of a pattern of a fourth embodiment;
Fig. 11 is a schematic view of a pattern of a fifth embodiment;
Fig. 12 is a schematic view of a pattern of a sixth embodiment;
Fig. 13 is a schematic view of a pattern of a seventh embodiment;
Fig. 14 is a schematic view of a pattern of an eighth embodiment;
Fig. 15 is a schematic view of a pattern of a ninth embodiment;
Fig. 16 is a schematic view of the contact lens of a fourth embodiment according to the present invention;
Fig. 17 is a schematic view of the contact lens of a fifth embodiment according to the present invention;
Fig. 18 is a schematic view of the contact lens of a sixth embodiment according to the present invention;
Fig. 19 is a schematic view of the contact lens of a seventh embodiment according to the present invention;
Fig. 20 is a schematic sectional view of sub-patterns of a first embodiment;
Fig. 21 is a schematic sectional view of sub-patterns of a second embodiment;
Fig. 22 is a schematic sectional view of sub-patterns of a third embodiment;
Fig. 23 is a schematic sectional view of sub-patterns of a fourth embodiment;
Fig. 24 is a schematic sectional view of a first exemplified type of the contact lens;
Fig. 25 is a schematic sectional view of a second exemplified type of the contact lens;
Fig. 26 is a schematic sectional view of a third exemplified type of the contact lens;
Fig. 27 is a schematic sectional view of a fourth exemplified type of the contact lens;
Fig. 28 is a schematic sectional view of a fifth exemplified type of the contact lens;
Fig. 29 is a schematic diagram showing a simulated moire pattern;
Fig. 30 is a schematic block diagram of a detection device of a first embodiment according to the present invention;
Fig. 31 is a schematic block diagram of a detection device of a second embodiment according to the present invention;
Fig. 32 is a flow chart of a method for analyzing change of moire pattern of contact lens of a first embodiment;
Fig. 33 is a flow chart of step S40 of a first embodiment;
Fig. 34 is a flow chart of step S410 of a first embodiment;
Fig. 35 is a schematic diagram showing step S410 of an exemplified case;
Fig. 36 is a schematic diagram showing a processed image after rotating 45 times;
Fig. 37 is a view illustrating a use state of a system for monitoring an intraocular pressure variation of a first embodiment according to the present invention; and
Fig. 38 is a view illustrating a use state of a system for monitoring an intraocular pressure variation of a second embodiment according to the present invention.

### DETAILED DESCRIPTION

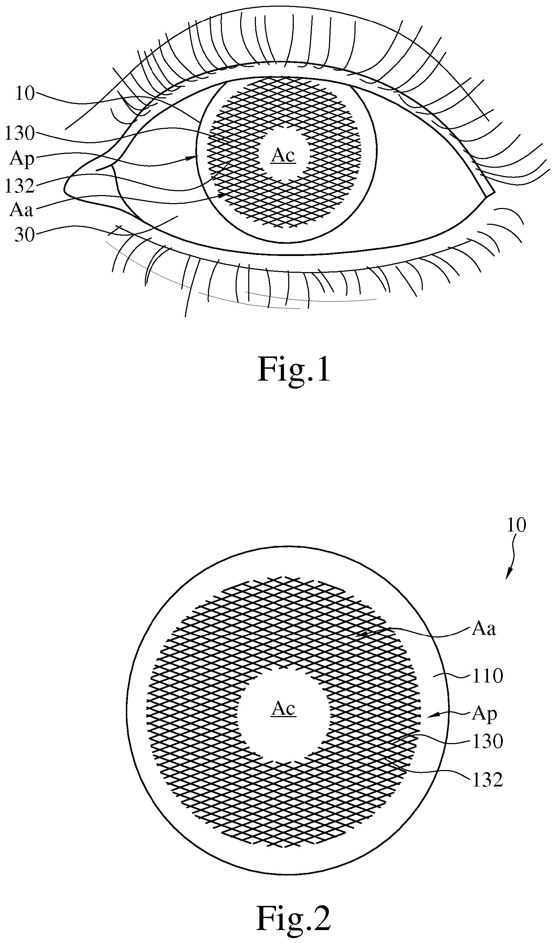

Please referring to Figs. 1-3, a contact lens without moire pattern in center (hereinafter called as the contact lens 10) is used for sensing change of an intraocular pressure (IOP). The contact lens 10 includes a first material layer 110 and a pattern (hereinafter referred to as a first pattern 130). Herein, the first material layer 110 is made of a light transmissive material. The first material layer 110 may be a rigid contact lens material or a soft contact lens material. The first pattern 130 is formed of a plurality of sub-patterns (hereinafter referred to as first sub-pattern) arranged at intervals.

From looking down the surface of the contact lens 10, the contact lens 10 has a central region Ac and an annular region Aa around the central region Ac. When the contact lens 10 is placed on a surface of an eyeball 30 of a user, the central region Ac corresponds to a pupil of the eyeball 30 and the annular region Aa corresponds to an iris of the eyeball 30. That is, the central region Ac covers the pupil of the eyeball 30, and the annular region Aa covers the iris of the eyeball 30.

In some embodiments, the whole of the annular region Aa is disposed over the iris of the eyeball 30 when the contact lens 10 is normally worn on the eyeball 30 of the user. In other embodiments, a portion of the annular region Aa is disposed over the iris of the eyeball 30 and the other portion of the annular region Aa is disposed over a sclera of the eyeball 30 when the contact lens 10 is normally worn on the eyeball 30 of the user.

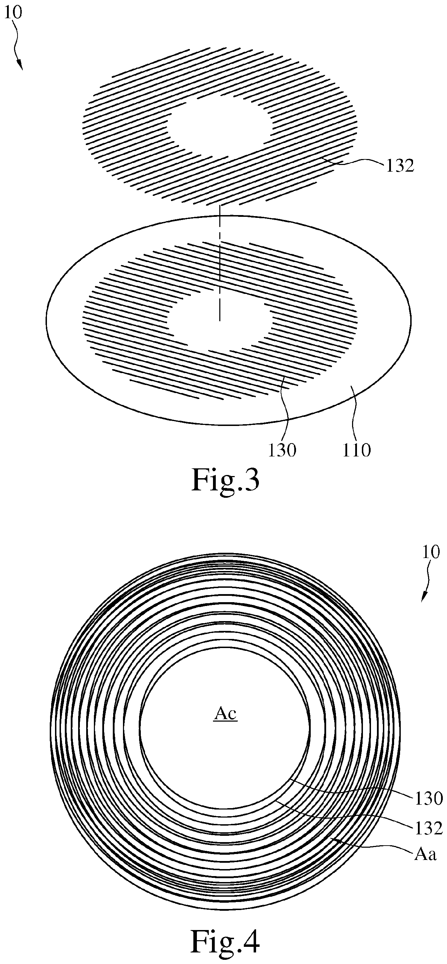

For example, the contact lens 10 may be divided into two regions (i.e. the central region Ac and the annular region Aa, as shown in Fig. 4) or three regions (i.e. the central region Ac, the annular region Aa and a periphery region Ap around the annular region Aa, as shown in Fig. 2).

In some embodiments, the annular region Aa may not cover the pupil of the eyeball 30 when the contact lens 10 is normally worn on the eyeball 30 of the user.

In some embodiments, the size of the central region Ac is equal to or larger than the size of the pupil of the eyeball 30. In one exemplified case, the central region Ac may cover whole the pupil of the eyeball 30. In another exemplified case, the central region Ac may cover whole the pupil of the eyeball 30 and a portion of the iris of the eyeball 30 near the pupil.

The first material layer 110 has the same region distribution as the contact lens 10. That is, the first material layer 110 has the central region Ac and the annular region Aa around the central region Ac.

The first pattern 130 is formed on the first material layer 110. When the first pattern 130 overlaps with another pattern (hereinafter referred to as a second pattern 132), a moire pattern is formed. The second pattern 132 is also formed of a plurality of sub-patterns (hereinafter referred to as second sub-pattern) arranged at intervals.

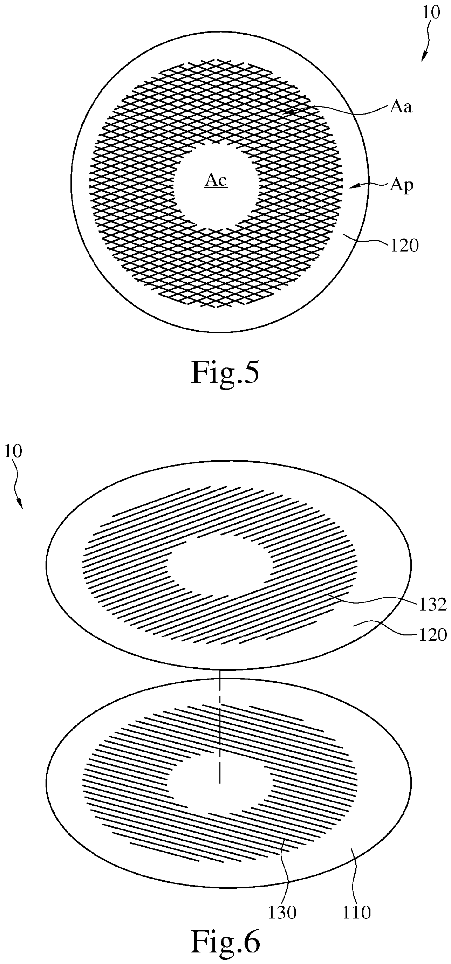

The second pattern 132 is formed on a second material layer 120, and the second material layer 120 is disposed on the first material layer 110, as shown in Figs. 5-6. Herein, the second material layer 120 is made of a light transmissive material. The second material layer 120 may be a rigid contact lens material or a soft contact lens material.

In some embodiments, the second pattern 132 is projected onto the first material layer 110 by a projection unit (not shown), as shown in Figs. 2-3.

In some embodiments, the moire pattern is an overlapping pattern of the first pattern 130 and the second pattern 132. In other embodiments, the moire pattern is an image of the first pattern 130 imaged on one surface of the second material layer 120 through the second pattern 132 on another surface of the second material layer 120.

Each aforementioned pattern 13 (i.e. any of the first pattern 130 and the second pattern 132) is formed of a plurality of sub-patterns 13a, 13b, 13c, 13d, 13e (i.e. the first sub-patterns or the second sub-patterns) arranged at intervals.

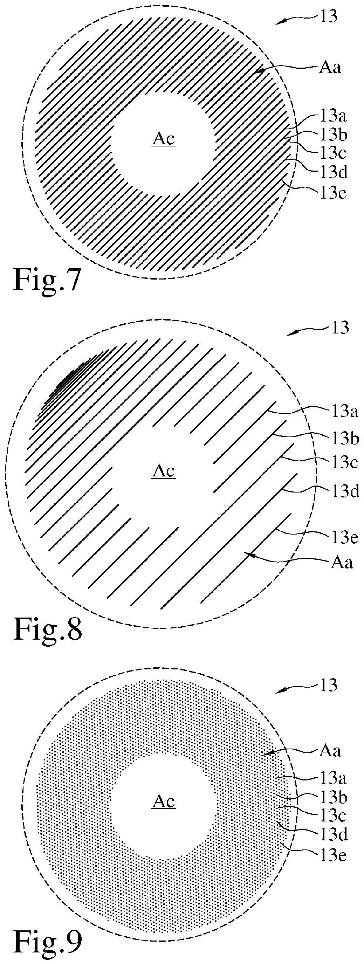

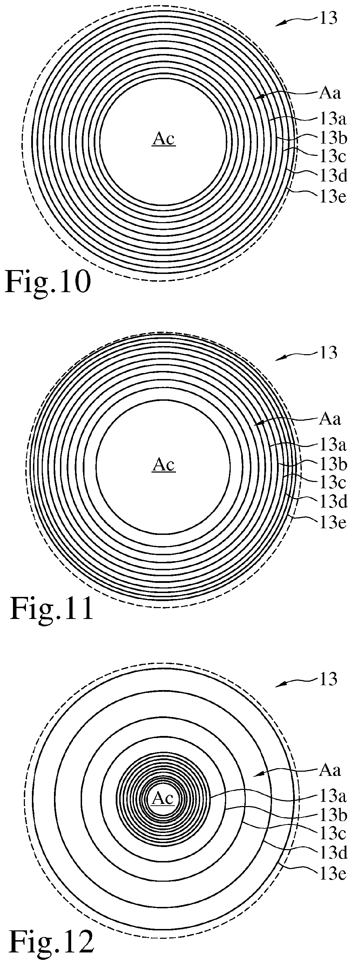

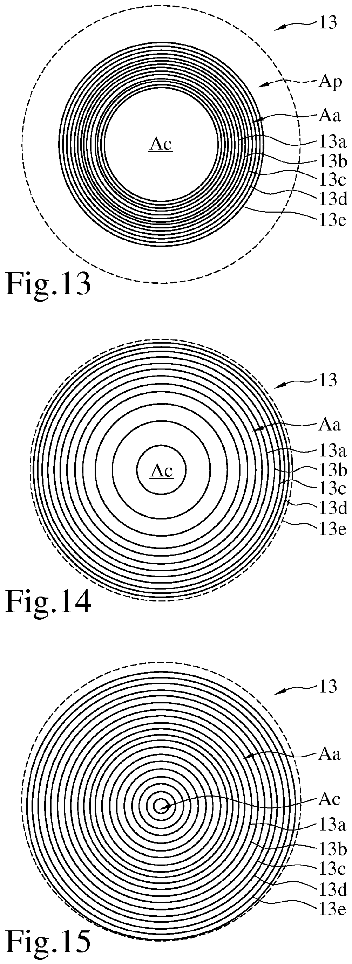

In some embodiments, the sub-patterns 13a, 13b, 13c, 13d, 13e of the pattern 13 may be straight lines (or strips) arranged at intervals (as shown in Figs. 7-8), points arranged at intervals (as shown in Fig. 9), circles arranged at intervals (as shown in Figs. 10-15), ellipses arranged at intervals, triangles arranged at intervals, rectangles arranged at intervals, or any other polygons arranged at intervals.

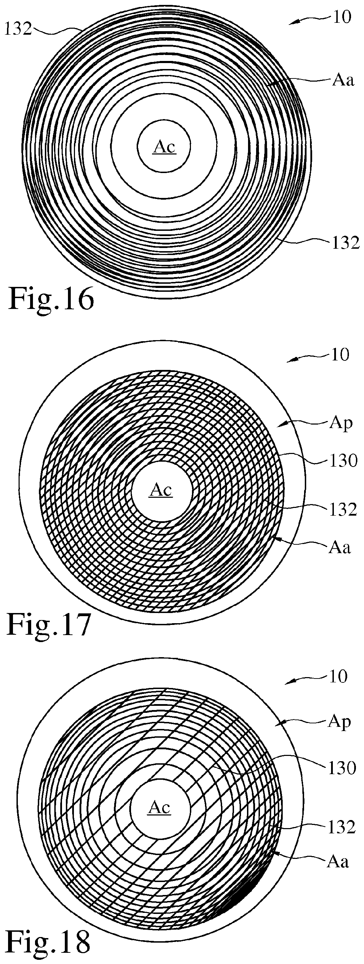

In some embodiments, the first pattern 130 and the second pattern 132 are the same patterns with a shift. For example, in one exemplified case, the first pattern 130 and the second pattern 132 both are concentric circles, and circle center of the first pattern 130 and circle center of the second pattern 132 are different in position, as shown in Fig. 4. In another exemplified case, the first pattern 130 and the second pattern 132 both are straight lines, and extension direction of the first pattern 130 is different from that of the second pattern 132, as shown in Fig. 2. Herein, the same patterns (the first pattern 130 and the second pattern 132) may have the same number of the sub-patterns (as shown in Fig. 4), or have the different number of the sub-patterns (as shown in Fig. 16).

In other embodiments, the first pattern 130 and the second pattern 132 are the different patterns in shape, intervals, or combination thereof, as shown in Figs. 17-19.

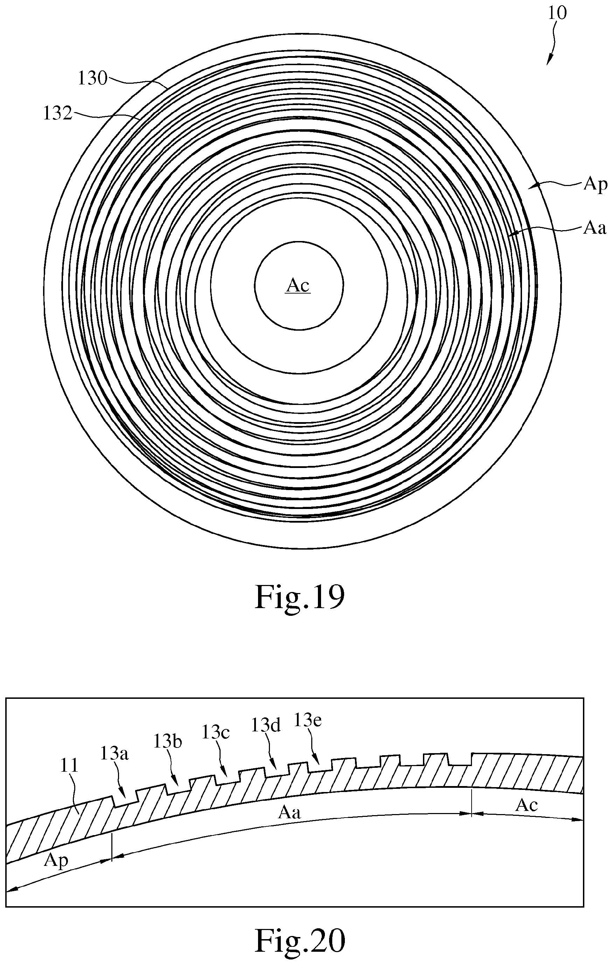

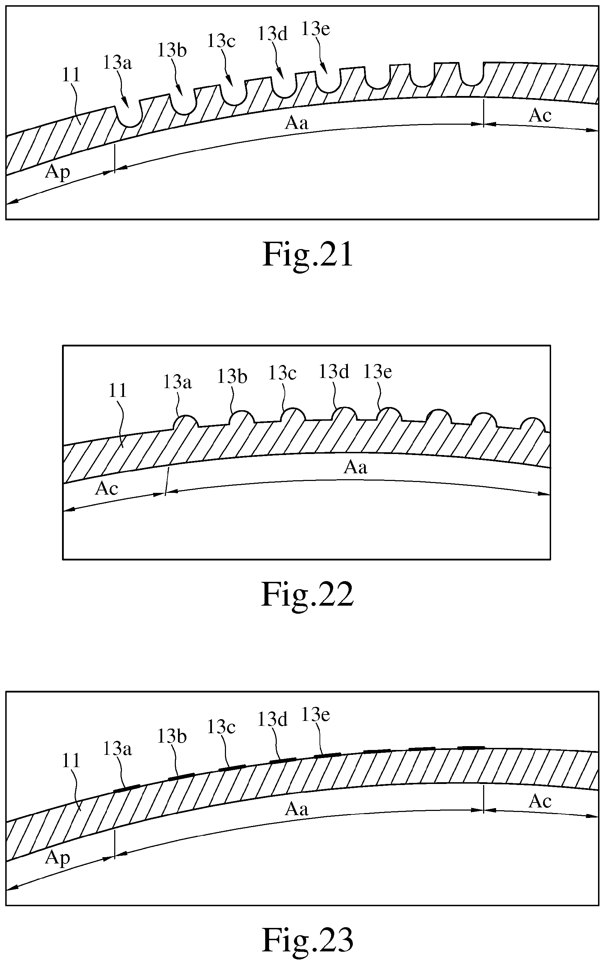

In some embodiments, in the same pattern 13, the sub-patterns 13a, 13b, 13c, 13d, 13e are grooves formed on the surface of the light transmissive material layer 11 (i.e. any of the first material layer 110 and the second material layer 120), as shown in Figs. 20-21. Herein, a bottom of each groove is a flat surface (as shown in Fig. 20) or an arc surface (as shown in Fig. 21). In other words, the pattern 13 is presented macroscopically by the grooves. Herein, the grooves with the arc surfaces act as convex lenses or concave lenses. In a particular embodiment, the bottom of each groove of the first pattern 130 is the flat surface.

In other embodiments, in the same pattern 13, the sub-patterns 13a, 13b, 13c, 13d, 13e are protrusions formed on the surface of the light transmissive material layer 11 (i.e. any of the first material layer 110 and the second material layer 120), as shown in Fig. 22. In other words, the pattern 13 is presented macroscopically by the protrusions. Herein, the protrusions act as convex lenses or concave lenses.

In yet other embodiments, in the same pattern 13, the sub-patterns 13a, 13b, 13c, 13d, 13e are prints applied to or printed on the surface of the light transmissive material layer 11 (i.e. any of the first material layer 110 and the second material layer 120), as shown in Fig. 23. In other words, the sub-patterns 13a, 13b, 13c, 13d, 13e of the pattern 13 are the prints, such as a pigment or dye.

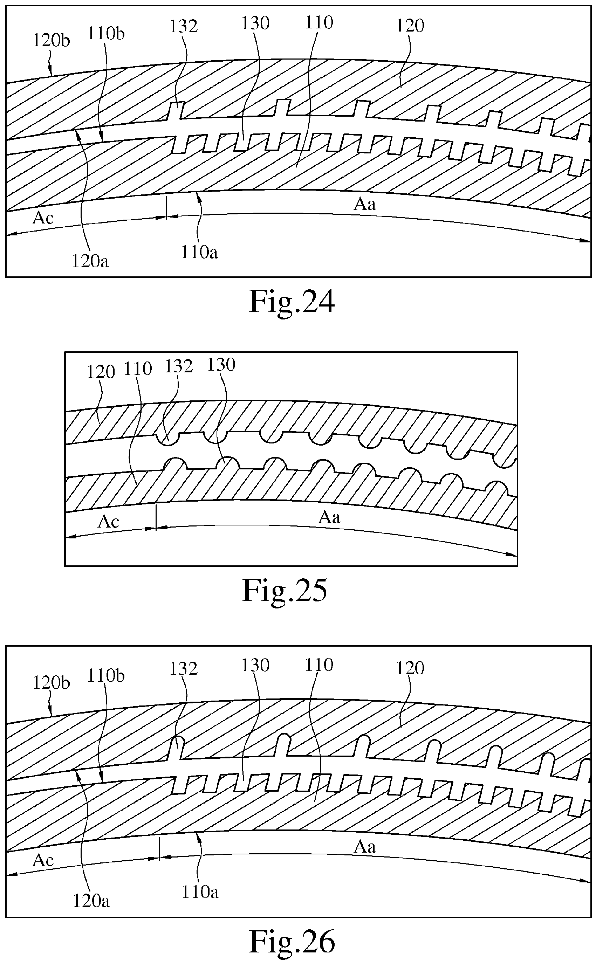

In some embodiments, the type of the first sub-patterns of the first pattern 130 and the type of the second sub-patterns of the second pattern 132 are the same, such as both grooves with the same bottom surfaces (as shown in Fig. 24), both protrusions (as shown in Fig. 25), or both prints (not shown).

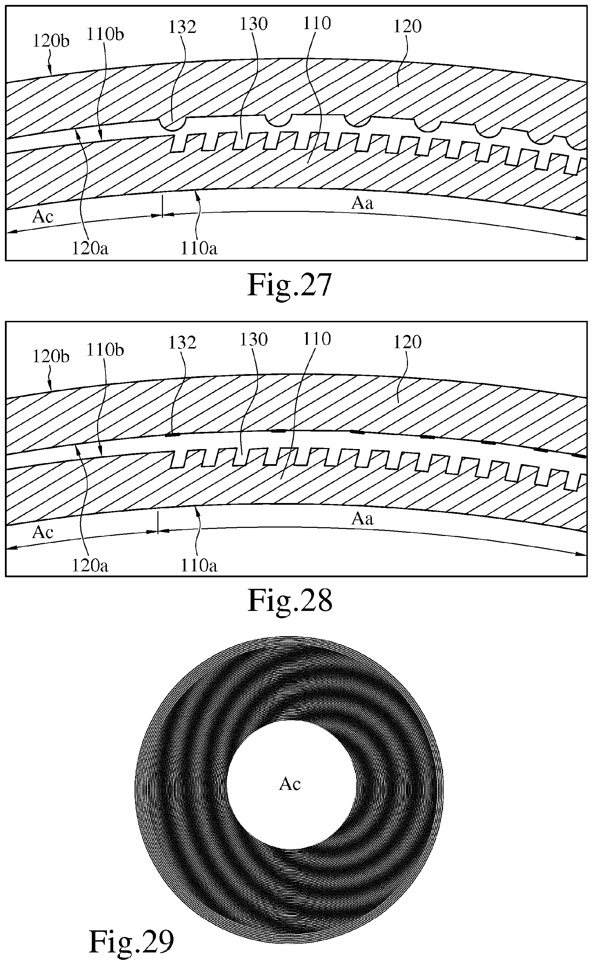

In other embodiments, the type of the first sub-patterns of the first pattern 130 and the type of the second sub-patterns of the second pattern 132 are different. For example, in first exemplified case, the first sub-patterns of the first pattern 130 and the second sub-patterns of the second pattern 132 are the grooves with different bottom surfaces, as shown in Fig. 26. In second exemplified case, the first sub-patterns of the first pattern 130 and the second sub-patterns of the second pattern 132 are the grooves and the protrusions, as shown in Fig. 27. In third exemplified case, the first sub-patterns of the first pattern 130 and the second sub-patterns of the second pattern 132 are the grooves and the prints, as shown in Fig. 28. In fourth exemplified case, the first sub-patterns of the first pattern 130 and the second sub-patterns of the second pattern 132 are the grooves and the projected image (not shown).

In some embodiments, in the same pattern 13, the intervals between the sub-patterns 13a, 13b, 13c, 13d, 13e are the same, as shown in Figs. 7, 9, 10, 13 and 15. In other embodiments, in the same pattern 13, the intervals between the sub-patterns 13a, 13b, 13c, 13d, 13e are different (as shown in Figs. 8, 11, 12 and 14), such as gradually decreased or randomly changed.

Please refer to Figs. 24-28, in some embodiments, when the first material layer 110 has two surfaces (hereinafter referred to as a first surface 110a and a second surface 110b), the first surface 110a and the second surface 110b opposite each other. In some embodiments, the first pattern 130 is formed in the annular region Aa at the second surface 110b. And, when the user wears the contact lens 10, the first surface 110a contacts the eyeball of the user.

In some embodiments, if the contact lens 10 has the second material layer 120, the second material layer 120 is connected to the first material layer 110. For example, an edge of the second material layer 120 is jointed with an edge of the first material layer 110.

Here, the second material layer 120 has two surfaces (hereinafter referred to as a first surface 120a and a second surface 120b), and the first surface 120a and the second surface 120b opposite each other. The first surface 120a of the second material layer 120 is connected to the second surface 110b of the first material layer 110. In some embodiments, the second pattern 132 is formed in the first surface 120a.

The second material layer 120 has the same region distribution as the contact lens 10. That is, the second material layer 120 has the central region Ac and the annular region Aa around the central region Ac. In some embodiments, the second pattern 132 is formed in the annular region Aa at the first surface 120a.

A buffer layer (not shown) is sandwiched between the first material layer 110 and the second material layer 120. The buffer layer may be a solution with biocompatibility, such as oil, hydrogel, a borate buffer solution, or a phosphate buffer solution, or may be air.

In some embodiments, not covered by the claims, if the contact lens 10 does not have the second material layer 120 (i.e. the second pattern 132 is a projected image), the contact lens 10 may further include a planar layer (not shown), and the planar layer is disposed on the second surface 110b of the first material layer 110.

Here, the contact lens 10 has curvature, so that when the user wears the contact lens 10, the first material layer 110 is adhered to the eyeball of the user. Therefore, the curvature of the first material layer 110 is changed as the cornea curvature changes, so that a period of the first pattern 130 is changed accordingly and then the period of the moire pattern is also changed.

In a simulated experiment, the first pattern 130 and the second pattern 132 are both the patterns 13 showed in Fig. 10, and there is a shift between the first pattern 130 and the second pattern 132. Accordingly, the moire pattern as shown in Fig. 29 is obtained.

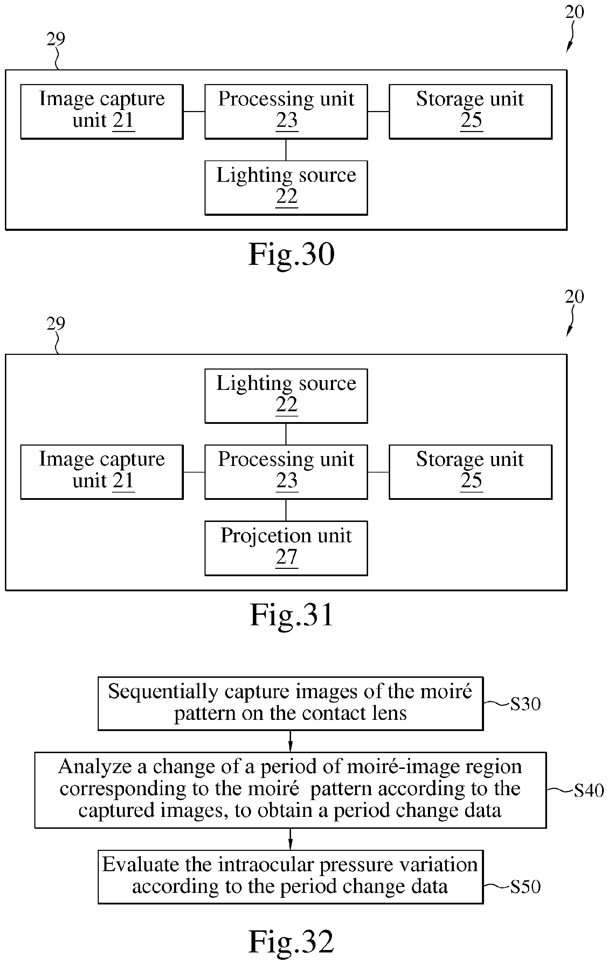

In some embodiments, please referring to Figs. 30-31, the contact lens 10 may be used in combination with a detection device 20, so that the detection device 20 execute a monitoring procedure to provide long-term and real-time intraocular pressure monitoring when the user wears the contact lens 10.

If the contact lens 10 has the first pattern 130 and the second pattern 132, the detection device 20 includes an image capture unit 21 and a processing unit 23, as shown in Fig. 30. The image capture unit 21 is electrically connected to the processing unit 23.

If, in embodiments not covered by the claims, the contact lens 10 has the first pattern 130 but does not have the second pattern 132, the detection device 20 includes an image capture unit 21, a processing unit 23 and a projection unit 27, as shown in Fig. 31. The image capture unit 21 is electrically connected to the processing unit 23, and the projection unit 27 is electrically connected to the processing unit 23. Under the control of the processing unit 23, the projection unit 27 projects the second pattern 132 on the contact lens 10 when the detection device 20 monitors an intraocular pressure variation of the user. The second pattern 132 projected by the projection unit 27 overlaps with the first pattern 130 in the contact lens 10 to form the moire pattern.

As to the monitoring procedure, referring to Figs. 30-32, the image capture unit 21 sequentially captures images of the moire pattern on the contact lens 10 according to a time interval, to sequentially obtain a plurality of images corresponding to the moire pattern on the contact lens 10 (Step S30), and the processing unit 23 receives the captured images from the image capture unit 21. Herein, each captured image has a moiré-image region exhibiting the moire pattern and an iris-image region exhibiting the iris of the eyeball 30.

Then, the processing unit 23 analyzes a change of a period of the moire-image region corresponding to the moire pattern according to the captured images, to obtain a period change data (Step S40). And, the processing unit 23 evaluates the intraocular pressure variation according to the period change data (Step S50).

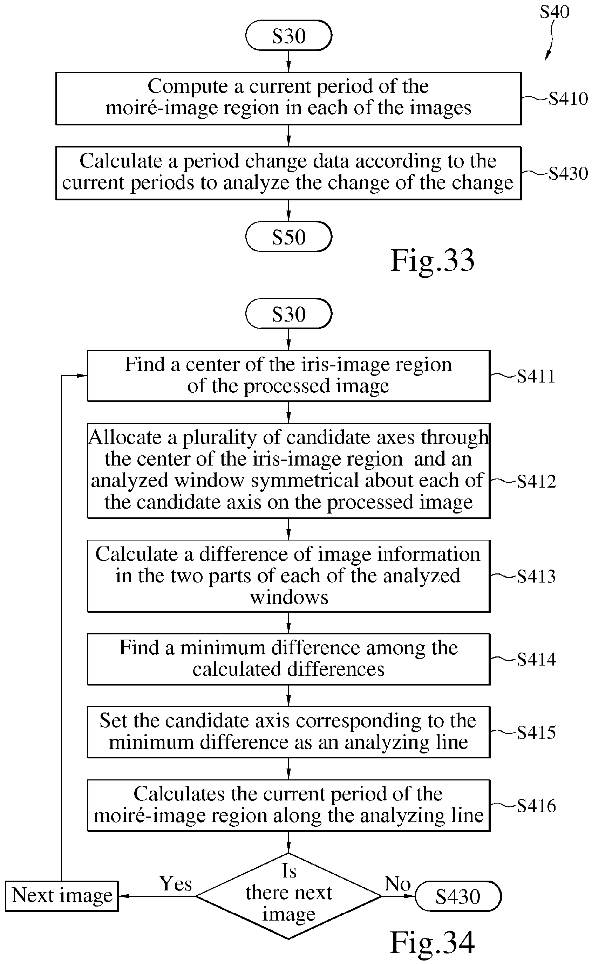

In some embodiments of the S40, referring to Figs. 30-33, the processing unit 23 computes a current period of the moire-image region corresponding to the moire pattern in each of the images (Step S410) and calculates a period change data according to the current periods to analyze the change of the change (Step S430).

In some embodiments of the S410, referring to Figs. 30-34, the processing unit 23 processes each image to obtain the current period of the moire-image region in the processed image. For each of the images, the processing unit 23 finds a center of the iris-image region of the processed image (Step S411), and allocates a plurality of candidate axes through the center of the iris-image region and an analyzed window symmetrical about each of the candidate axis on the processed image (Step S412). Herein, each of the analyzed windows is divided into two parts by the corresponding candidate axis. Then, the processing unit 23 calculates a difference of image information in the two parts of each of the analyzed windows (Step S413), and finds a minimum difference among the calculated differences (Step S414). After the step S414, the processing unit 23 sets the candidate axis corresponding to the minimum difference as an analyzing line (Step S415), and calculates the current period of the moire-image region in the processed image along the analyzing line (Step S416).

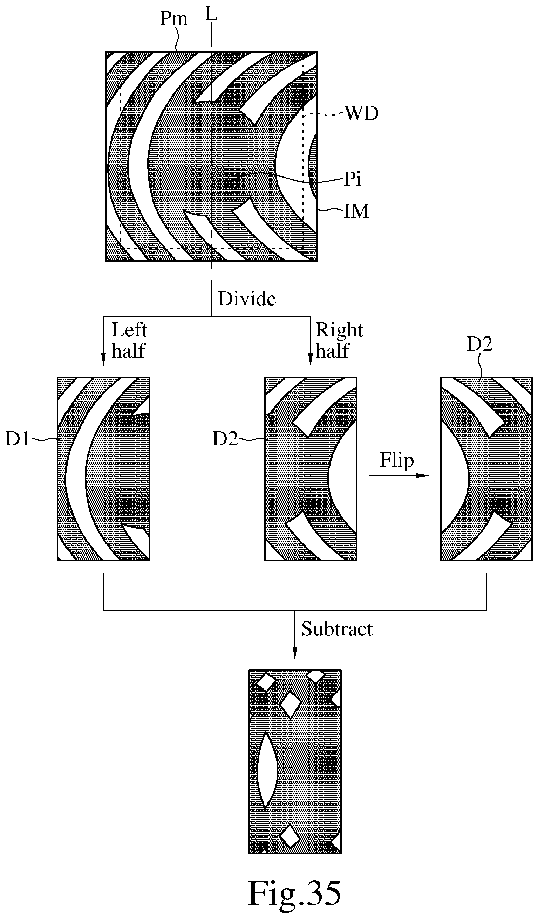

For example, referring to Fig. 35, the image capture unit 21 captures the image of the moire pattern on the contact lens 10 to obtain the captured image IM with the moire-image region Pm exhibiting the moire pattern and the iris-image region Pi exhibiting the iris of the eyeball 30. The processing unit 23 forms one candidate axe L through the center of the iris-image region Pi on the captured image IM, and forms one analyzed window WD symmetrical about the formed candidate axis L on the captured image IM. The analyzed window WD is divided into two parts D1, D2 by the candidate axis L. The processing unit 23 subtracts the image information of the captured image IM in one part of the analyzed window WD from the image information of the captured image IM in the other part of the analyzed window WD, to obtain the difference of the image information. The difference of the image information may be the resultant intensity.

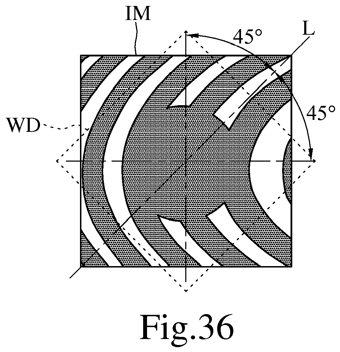

The processing unit 23 successively rotates a combination of the formed analyzed window WD and the formed candidate axis L around the center of the iris-image region Pi with respect to the captured image IM a plurality of times, and executes the aforementioned steps again after each rotation, to obtain the differences of the image information under the different total rotation angles. The total rotation angle is from 0 degree to 180 degree. For example, if a rotation angle unit is 1 degree and the rotation time is 45, the total rotation angle is 45 degree and the captured image IM with the analyzed window WD and the candidate axis L is as shown in Fig. 36.

In some embodiments, the captured image IM may be transformed in Fourier domain for spatial frequency analysis to obtain the current period of the moire-image region Pm corresponding to the moire pattern.

In some embodiments, the detection device 20 may further include a storage unit 25. The storage unit 25 is electrically connected to the processing unit 23.

The storage unit 25 stores data information for the monitoring procedure.

In some embodiments, the data information may include the correspondence between the period change data and the intraocular pressure variation. And, the processing unit 23 may convert each calculated period change data into the intraocular pressure variation according to the stored data information.

In some embodiments, the storage unit 25 may further store a threshold. And, the processing unit 23 can determine whether the intraocular pressure variation of the user is normal according to the threshold and the intraocular pressure variation.

In some embodiments, the initial period of the moire-image region Pm corresponding to the moire pattern may be pre-stored in the storage unit 25. And, the processing unit 23 can calculating the period change data according to each current period and the initial period.

In some embodiments, if the contact lens 10 has the first pattern 130 but does not have the second pattern 132, the second pattern 132 may be pre-stored in the storage unit 25. When the detection device 20 monitors the intraocular pressure variation of the user, the processing unit 23 reads out the second pattern 132 from the storage unit 25 and controls the projection unit 27 to project the second pattern 132.

In some embodiments, the detection device 20 may further include a lighting source 22. The lighting source 22 is electrically connected to the processing unit 23. The lighting source 22 can provide enough brightness for the moire pattern, facilitating image capturing of the image capture unit 21.

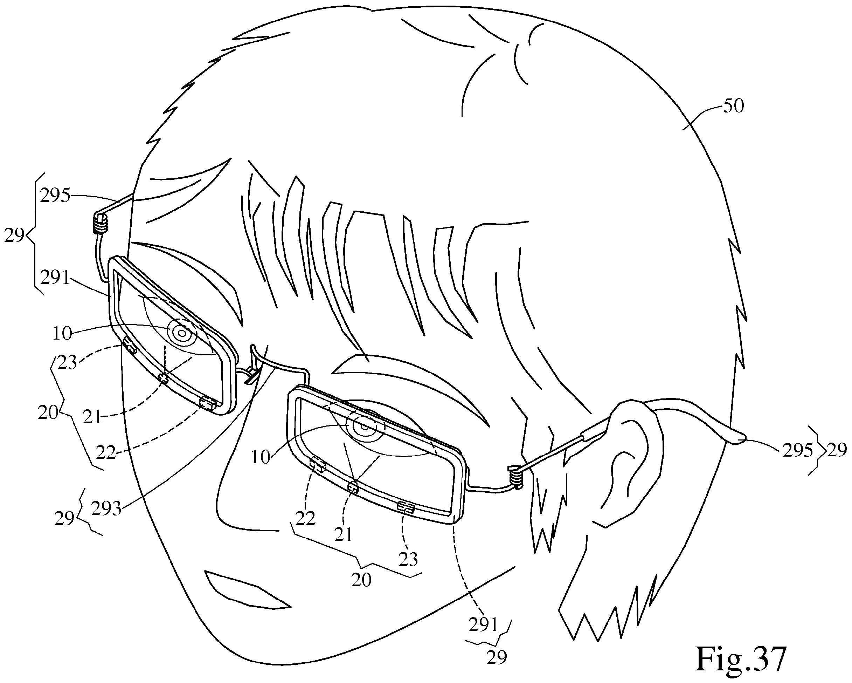

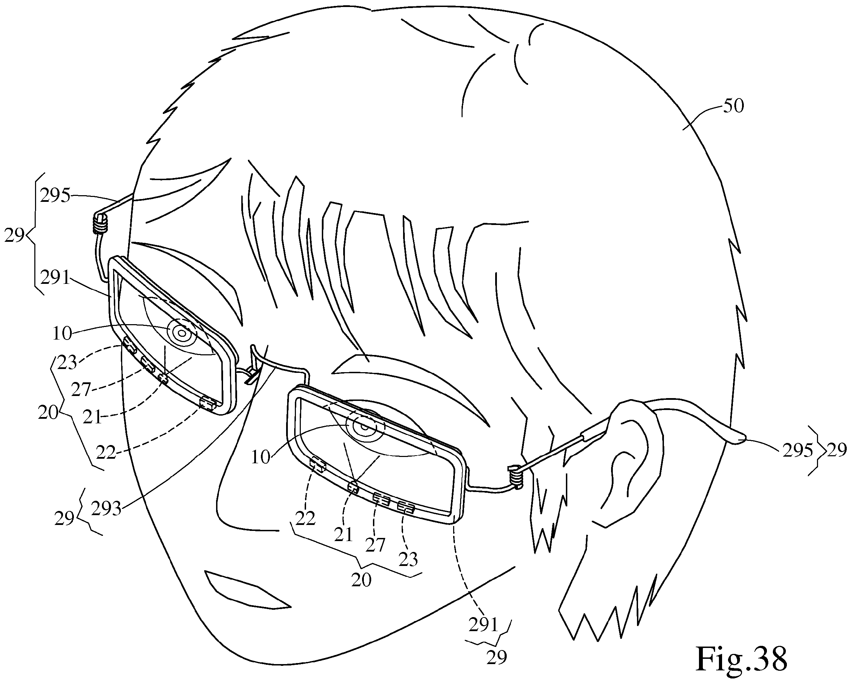

In some embodiments, referring to Figs. 30, 31, 37 and 38, the detection device 20 may further include a carrier 29. The carrier 29 may be formed as glasses. To execute the monitoring procedure, the user 50 may wear the carrier 29 and the contact lens 10 at the same time, so as to achieve long-term monitoring of the intraocular pressure variation of the user 50 at any time and any place.

The processing unit 23 and the storage unit may be disposed inside the carrier 29. The image capture unit 21 is embedded at an inner side of the carrier 29.

For example, the carrier 29 is the glasses, and the glasses include two lens frames 291, a connection frame 293, and two hanger frames 295. First sides of outer edges of the two lens frames 291 are connected to two ends of the connection frame 293 respectively, and each of second sides of the outer edges of the two lens frames 291 is connected to one end of each of the two hanger frames 295. Additionally, when the user 50 wears the glasses, the other end of each hanger frame 295 extends towards the user 50. The first side of the outer edge of each lens frame 291 is opposite the second side of the outer edge. Here, an inner edge of each lens frame 291 refers to an edge for mounting a common lens, and the outer edge is opposite the inner edge.

The image capture unit 21 is embedded at an inner side of the lens frame 291, that is, a side where the lens frame 291 extends towards the hanger frame 295. Additionally, when the user 50 wears the glasses, a capturing lens of the image capture unit 21 faces the iris of the eyeball 30 of the user 50, the pupil of the eyeball 30 of the user 50 or combination thereof.

If the detection device 20 has a projection unit 27, the projection unit 27 is disposed on the carrier 29 in a manner approximately same as the disposing manner of the image capture unit 21, so that the projection unit 27 can project the second pattern 132 onto the first pattern 130 in the optical region 112 of the contact lens 10.

If the detection device 20 has a lighting source 22, the lighting source 22 is disposed on the carrier 29 in a manner approximately same as the disposing manner of the image capture unit 21, so that the lighting source 22 can provide light rays required by the image capture unit 21 to capture the image.

As above, according to the embodiments, the contact lens 10 is designed to appear a moire pattern in the annular region of the surface thereof, and not appear the moire pattern in the central region corresponding to the viewing field (i.e. the pupil of the eyeball 30). The contact lens 10 is configured to sense the curvature of the cornea of the eyeball 30 of the user 50, such that the change of the moire pattern is directly related to the IOP variation. Therefore, the contact lens 10 does not affect the viewing transparency for the user 50 who wears it for continual the IOP monitoring, and the amount of data to be processed for obtaining the corresponding IOP variation is significantly reduced. According to some embodiments, the change of the cornea of the eyeball 30 of the user 50 is accurately measured without need of dropping fluorescent agents, thereby avoiding damage or infection to the cornea. According to some embodiments, the structure (e.g. the grooves or the protrusions) or the prints of the pattern and the main body (i.e., the light transmissive material layer) of the contact lens 10 are integrally formed, and the pattern (i.e. the first pattern 130 and the second pattern 132) of the contact lens 10 do not contact the eyeball, thereby not affecting the wearing comfort.

The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

## Claims

1. A contact lens without moire pattern in center, comprising:
a first material layer (110), having a central region (Ac) and an annular region (Aa) around the central region (Ac), wherein when the contact lens (10) is placed on a surface of an eyeball (30), the central region (Ac) corresponds to a pupil of the eyeball (30) and the annular region (Aa) corresponds to an iris of the eyeball (30); and
a first pattern (130), formed in the annular region (Aa), formed of a plurality of first sub-patterns (13a, 13b, 13c, 13d, 13e) arranged at intervals, configured to overlap with a second pattern (132) to form a moire pattern, wherein the first pattern (130) is not formed in the central region (Ac);
a second material layer (120), located on the first material layer (110);
wherein the second pattern (132) is formed on the second material layer (120) and the second pattern (132) is formed of a plurality of second sub-patterns (13a, 13b, 13c, 13d, 13e) arranged at intervals; and
wherein a buffer layer is sandwiched between the first material layer (110) and the second material layer (120).

2. The contact lens according to claim 1, wherein the first sub-patterns (13a, 13b, 13c, 13d, 13e) are a plurality of grooves, a plurality of protrusions, or a plurality of prints.

3. The contact lens according to claim 1, wherein the moire pattern is an overlapping pattern of the first pattern (130) and the second pattern (132).

## Patentansprüche

1. Eine Kontaktlinse ohne Moire-Muster in der Mitte, aufweisend:
eine erste Material-Schicht (110), welche eine mittlere Region (Ac) und eine ringförmige Region (Aa) um die mittlere Region (Ac) herum hat, wobei wenn die Kontaktlinse (10) auf einer Oberfläche eines Augapfels (30) platziert ist, die mittlere Region (Ac) zu einer Pupille des Augapfels (30) korrespondiert und die ringförmige Region (Aa) zu einer Iris des Augapfels (30) korrespondiert; und
ein erstes Muster (130), welches in der ringförmigen Region (Aa) gebildet ist, welches aus einer Mehrzahl an ersten Unter-Mustern (13a, 13b, 13c, 13d, 13e) gebildet ist, welche in Abständen angeordnet sind, welches konfiguriert ist, um mit einem zweiten Muster (132) zu überlappen, um ein Moire-Muster zu bilden, wobei das erste Muster (130) nicht in der mittleren Region (Ac) gebildet ist;
eine zweite Material-Schicht (120), welche auf der ersten Material-Schicht (110) positioniert ist;
wobei das zweite Muster (132) auf der zweien Material-Schicht (120) gebildet ist und das zweite Muster (132) aus einer Mehrzahl an zweiten Unter-Mustern (13a, 13b, 13c, 13d, 13e) gebildet ist, welche in Abständen angeordnet sind; und
wobei eine Puffer-Schicht in einer Sandwich-Position zwischen der ersten Material-Schicht (110) und der zweiten Material-Schicht (120) ist.

2. Eine Kontaktlinse nach Anspruch 1, wobei die ersten Unter-Mustern (13a, 13b, 13c, 13d, 13e) eine Mehrzahl an Schlitzen, eine Mehrzahl an Vorsprüngen oder eine Mehrzahl an Drucken sind.

3. Eine Kontaktlinse nach Anspruch 1, wobei das Moire-Muster ein überlappendes Muster aus dem ersten Muster (130) und dem zweiten Muster (132) ist.

## Revendications

1. Lentille de contact sans motif de moiré au centre, comprenant :
une couche d'un premier matériau (110), présentant une région centrale (Ac) et une région annulaire (Aa) autour de la région centrale (Ac), dans laquelle, lorsque la lentille de contact (10) est placée sur la surface d'un globe oculaire (30), la région centrale (Ac) correspond à la pupille du globe oculaire (30), et la région annulaire (Aa) correspond à l'iris du globe oculaire (30) ; et
un premier motif (130), formé dans la région annulaire (Aa), constitué d'une pluralité de premiers motifs secondaires (13a, 13b, 13c, 13d, 13e) agencés à intervalles, configuré pour se superposer à un second motif (132) afin de former un motif de moiré, dans laquelle le premier motif (130) n'est pas formé dans la région centrale (Ac) ;
une couche d'un second matériau (120), située sur la couche d'un premier matériau (110) ;
dans laquelle le second motif (132) est formé sur la couche d'un second matériau (120), et le second motif (132) est constitué d'une pluralité de seconds motifs secondaires (13a, 13b, 13c, 13d, 13e) agencés à intervalles ; et
dans laquelle une couche tampon est prise en sandwich entre la couche d'un premier matériau (110) et la couche d'un second matériau (120).

2. Lentille de contact selon la revendication 1, dans laquelle les premiers motifs secondaires (13a, 13b, 13c, 13d, 13e) sont constitués par une pluralité de rainures, une pluralité de saillies, ou une pluralité d'impressions.

3. Lentille de contact selon la revendication 1, dans laquelle le motif de moiré est un motif constitué par la superposition du premier motif (130) et du second motif (132).
